# EUROPEAN PATENT APPLICATION

(11) **EP 3 875 595 A1**
(43) Date of publication of application: **08.09.2021**
(21) Application number: 19878782.2
(22) Date of filing: 01.11.2019
(51) Int. Cl.: C12P 21/08, C12N 5/16, C12N 1/00

(54) **LIQUID MEDIUM PREPARATION METHOD**

(30) Priority: 02.11.2018 US 201862754793 P
(71) Applicant: Kyowa Kirin Co., Ltd., Tokyo 100-0004 (JP)
(72) Inventor: HIRA Shusuke, Tokyo 100-0004 (JP); SUZAWA Toshiyuki, Tokyo 100-0004 (JP)
(74) Representative: Hoffmann Eitle
(86) International application number: PCT/JP2019/043031
(87) International publication number: WO 2020/091041

(57) **Abstract**

The invention relates to a method for preparing a liquid medium which is characterized by dissolving a desired component efficiently by controlling an amount of supplied oxygen, a liquid medium prepared by the preparation method, a method for culturing cells using the liquid medium prepared by the preparation method, a method for producing a physiologically active substance having desired quality using the culture method, and a physiologically active substance having desired quality produced by using the production method.

## Description

### Technical Field

The present invention relates to a method for preparing a liquid medium, a liquid medium prepared by the preparation method, a method for culturing cells using the liquid medium prepared by the preparation method, a method for producing a polypeptide having desired quality using the culture method and a polypeptide having desired quality produced using the production method.

### Background Art

Various biopharmaceuticals containing a physiologically active substance, especially a polypeptide, a glycoprotein or an antibody, as an active ingredient have been approved recently, and many candidate substances are under development.

It is a problem to be solved in the pharmaceutical industry that the production costs of biopharmaceuticals are higher than those of small-molecule drugs. So far, efforts to improve the productivity of biopharmaceuticals and to reduce the costs have been made through improvement of the liquid medium used for cell culture, such as reinforcement of a part of the components, addition of a new component and an increase in the concentration. It is necessary that a liquid medium used for cell culture contains sufficient amounts of nutrients which are required for the cell growth and for the production of a physiologically active substance as the active ingredient of a biopharmaceutical. In general, a liquid medium used for cell culture contains amino acids, nucleic acids, saccharides, lipids, vitamins, metals, inorganic salts, derivatives thereof and the like as components. A liquid medium is used for cells in a state in which the components are dissolved in the liquid medium and used for the growth and the survival of the cells and for the production of a physiologically active substance.

It is known that the composition of the liquid medium used for cell culture or a part of the components affects the quality of the active ingredient of the produced biopharmaceutical. In order to satisfy the aimed quality as a biopharmaceutical, identification of the medium components which affect the quality of the active ingredient of the biopharmaceutical or optimization of the composition is important for the development of a liquid medium. In particular, it is reported that, while the amounts of metals contained in a liquid medium used for cell culture are very low, the metals affect the productivity or the quality of the produced physiologically active substance (NPL 1). For example, it is known that the concentration of copper contained in a liquid medium used for cell culture affects the cell growth, the productivity or the quality of the produced antibody (NPL 2).

Under the circumstances, demand for preparing an optimum liquid medium for producing physiologically active substances is increasing in order to improve the productivity of biopharmaceuticals and to produce biopharmaceuticals having desired quality.

At the same time, for the production of a biopharmaceutical, it is required to secure the sterility of the medium used for cell culture to appropriately conduct culturing using cells and to secure the safeness of the product. Therefore, when a liquid medium is used for culture, the sterility is generally secured, for example by filtration through a membrane having a pore size of 0.2 µm or the like. Recently, use of a filtration step using a 0.1 µm membrane for the purpose of not only removing microorganisms but also removing mycoplasmas has been recommended, and membrane filters which can remove potential viruses have also been developed (NPL 3).

### Citation List

### Non-Patent Document

NPL 1: D. Bruhlmann et al., Biotechnology Progress, 2015, 31, 615-629
NPL 2: T. Kaschak et al., mAbs, 2011, 6, 577-583
NPL 3: R. J. Hay et al., Nature, 1989, 339, 487-488

### Summary of Invention

### Technical Problem

A liquid medium is prepared by dissolving a powder medium in a solvent. When a part of the medium components is not dissolved in the solvent during the dissolution step, the undissolved components are removed in the filtration step. Accordingly, during the preparation step of a liquid medium, the medium components should be effectively dissolved in the solvent before the filtration step so that the composition of the medium does not change between before and after the filtration step.

The present inventors have found that the solubility of the medium components into the solvent decreases and that the components of the medium change between before and after the filtration especially when the liquid medium is prepared in a large scale. Moreover, the inventors have found for the first time that the amount of a variant, which is a polypeptide-derived impurity, increases when a polypeptide is produced using a liquid medium which has been prepared in a large scale.

That is, the invention relates to a method for preparing a liquid medium in which the medium components are dissolved effectively in the solvent, a liquid medium prepared by the preparation method, a method for culturing cells using a liquid medium prepared by the preparation method, a method for producing a polypeptide having desired quality using the culture method and a polypeptide having desired quality produced using the production method.

### Solution to Problem

The inventors have intensively investigated a method for preparing a medium containing desired amounts of components. As a result, the inventors have found that the medium components can be dissolved effectively in the solvent and that a liquid medium containing desired amounts of desired components can be prepared by controlling the oxygen concentration during the preparation of the medium in a step of preparing a liquid medium. The invention has been thus completed.

That is, the invention relates to the followings.
1. A method for preparing a liquid medium, comprising a step of dissolving a powder medium in a solvent in the presence of oxygen.
2. The method for preparing a liquid medium described in above 1, wherein the powder medium comprises copper or a derivative thereof.
3. The method for preparing a liquid medium described in above 2, wherein the copper or the derivative thereof is a simple substance, an oxide, or a salt of copper or a hydrate thereof.
4. The method for preparing a liquid medium described in above 2 or 3, wherein the copper or the derivative thereof is copper(II) sulfate pentahydrate, copper(II) chloride dihydrate, copper(I) chloride, copper(II) nitrate trihydrate or a mixture thereof.
5. The method for preparing a liquid medium described in any one of above 1 to 4, comprising a step of adding cysteine, cystine or a derivative thereof.
6. The method for preparing a liquid medium described in any one of above 1 to 5, comprising at least one operation selected from the following (i) and (ii):
   (i) an operation of sending a gas containing oxygen from the upper surface and bringing the gas into contact with the solvent; and
   (ii) an operation of sparging the gas containing oxygen into the solvent.
7. The method for preparing a liquid medium described in any one of above 1 to 6, comprising an operation of stirring the solvent.
8. The method for preparing a liquid medium described in any one of above 1 to 7, further comprising a membrane filtration step after the step of dissolving the powder medium in the solvent in the presence of oxygen.
9. The method for preparing a liquid medium described in above 8, wherein a copper concentration in the liquid medium does not substantially change between before and after the membrane filtration.
10. The method for preparing a liquid medium described in above 8 or 9, wherein a pore size of a membrane filter used for the membrane filtration is 1 nm to 100 µm.
11. The method for preparing a liquid medium described in any one of above 1 to 10, comprising a step of measuring a dissolved oxygen concentration in the solvent or the liquid medium.
12. The method for preparing a liquid medium described in any one of above 1 to 11, comprising a step of measuring the dissolved oxygen concentration in the solvent or the liquid medium before or during the preparation of the liquid medium.
13. The method for preparing a liquid medium described in any one of above 1 to 12, wherein the solvent contains oxygen in an amount of 20% or more, 30% or more, 40% or more, 50% or more, 60% or more, 70% or more, 80% or more, 85% or more, 90% or more, 91% or more, 92% or more, 93% or more, 94% or more, 95% or more, 96% or more, 97% or more, 98% or more, 99% or more, or 100% of a saturated dissolved oxygen during the preparation of the liquid medium.
14. The method for preparing a liquid medium described in any one of above 1 to 13, wherein the amount of the solvent is 10 to 10000 L.
15. A liquid medium prepared by the preparation method described in any one of above 1 to 14.
16. A method for culturing cells using the liquid medium described in above 15.
17. The method for culturing cells described in above 16, wherein the cells are cells that express a polypeptide.
18. The method for culturing cells described in above 17, wherein the polypeptide is an antibody.
19. The method for culturing cells described in any one of above 16 to 18, wherein the cells are animal cells.
20. The method for culturing cells described in above 19, wherein the animal cells are Chinese hamster ovary cells.
21. The method for culturing cells described in any one of above 16 to 20, wherein the cells are genetically modified cells.
22. A method for producing a polypeptide, comprising culturing cells that express a desired polypeptide in the liquid medium described in above 15, producing and accumulating the polypeptide in a culture and collecting the polypeptide from the culture.
23. The method for producing a polypeptide described in above 22, wherein the polypeptide is an antibody.
24. The method for producing a polypeptide described in above 22 or 23, wherein the cells are animal cells.
25. The method for producing a polypeptide described in above 24, wherein the animal cells are Chinese hamster ovary cells.
26. The method for producing a polypeptide described in any one of above 22 to 25, wherein the cells are genetically modified cells.
27. A polypeptide produced using the production method described in any one of above 22 to 26.
28. A method for controlling generation of a variant derived from a polypeptide, wherein the liquid medium described in above 15 is used in a step of culturing cells that express the polypeptide.
29. The method for controlling the generation of a variant described in above 28, wherein the polypeptide is an antibody.
30. The method for controlling the generation of a variant described in above 29, wherein the variant is an antibody in which C-terminal proline is amidated.
31. The method for controlling the generation of a variant described in any one of above 28 to 30, wherein the cells are animal cells.
32. The method for controlling the generation of a variant described in above 31, wherein the animal cells are Chinese hamster ovary cells.
33. The method for controlling the generation of a variant described in any one of above 28 to 32, wherein the cells are genetically modified cells.

### Advantageous Effects of Invention

Surprisingly, the present inventors have found for the first time that the oxygen concentration during the preparation of a medium can be controlled, that the medium components can be dissolved effectively in the solvent and that a liquid medium containing desired amounts of desired components can be prepared by including a step of dissolving a powder medium in a solvent in the presence of oxygen in a method for preparing a liquid medium for cell culture. The inventors have also found that a polypeptide having desired quality can be produced using the obtained liquid medium containing the desired amounts of the desired components for cell culture.

That is, by the invention, a method for preparing a liquid medium in which the medium components are dissolved effectively in the solvent, a liquid medium prepared by the preparation method, a method for culturing cells using a liquid medium prepared by the preparation method, a method for producing a polypeptide having desired quality using the culture method and a polypeptide having desired quality produced using the production method can be provided.

### Brief Description of Drawings

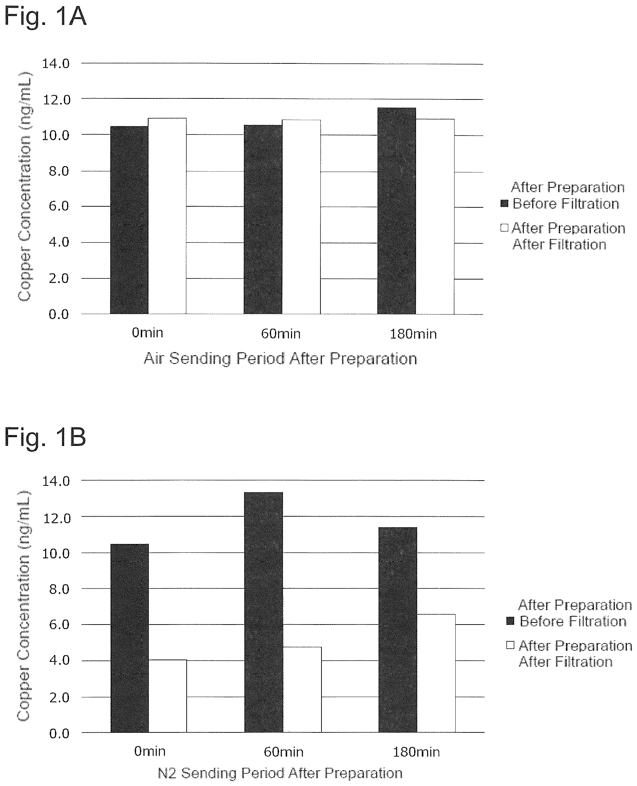

[Fig. 1A] Fig. 1A shows the copper concentrations in a medium prepared by adding water to a powder medium and then stirring for a certain period while sending air. In Fig. 1A, the black columns show the copper concentrations in the medium before filtration, and the white columns show the copper concentrations in the medium after filtration.
[Fig. 1B] Fig. 1B shows the copper concentrations in a medium prepared by adding water to a powder medium and then stirring for a certain period while sending nitrogen. In Fig. 1B, the black columns show the copper concentrations in the medium before filtration, and the white columns show the copper concentrations in the medium after filtration.

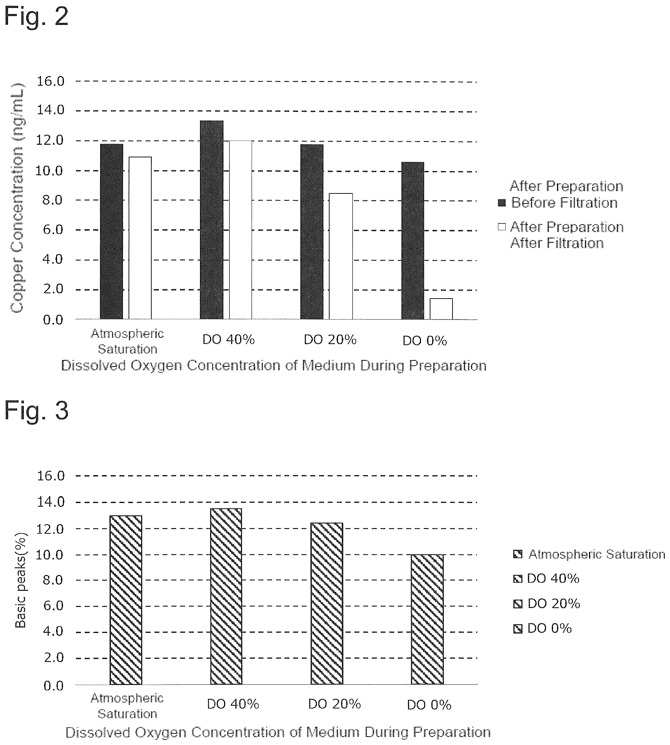

[Fig. 2] Fig. 2 shows the copper concentrations in liquid media prepared under different dissolved oxygen concentration conditions. The black columns show the copper concentrations in the media before filtration, and the white columns show the copper concentrations in the media after filtration.
[Fig. 3] Fig. 3 shows the percentages of the basic peaks of an antibody obtained by culturing antibody-producing cells using liquid media prepared under different dissolved oxygen concentration conditions.

### Description of Embodiments

The invention is explained in detail below. The invention relates to a method for preparing a liquid medium including a step of dissolving a powder medium in a solvent in the presence of oxygen.

The medium in the invention can be appropriately selected from commercially available media, and two or more kinds of medium may be mixed. Moreover, a known medium described in a document or the like can also be selected. A desired nutritional factor can be appropriately selected and added to such a medium. The medium may be composed of components appropriately selected from desired nutritional factors.

The medium in the invention may be any of a synthetic medium, a semisynthetic medium and a natural medium. Examples include a minimal essential medium, a serum-containing medium, a serum-free medium, a medium containing no animal-derived component, a protein-free medium, a completely synthetic medium and the like, and a serum-free medium, a protein-free medium or a completely synthetic medium is preferable.

Examples of the minimal essential medium in the invention include commercial media such as RPMI1640 medium [The Journal of the American Medical Association, 199, 519 (1967)], Eagle's MEM medium [Science, 122, 501 (1952)], Dulbecco's Modified MEM (DMEM) medium [Virology, 8, 396 (1959)], 199 medium [Proceeding of the Society for the Biological Medicine, 73, 1 (1950)], F12 medium (manufactured by LTI) [Proc. Natl. Acad. Sci. USA, 53, 288 (1965)], Iscove's Modified Dulbecco's medium (IMDM medium) [J. Experimental Medicine, 147, 923 (1978)], EX-CELL (registered trademark) 302 medium, EX-CELL (registered trademark) 325 medium (manufactured by SAFC Biosciences, Inc.) and CHO-S-SFMII medium (manufactured by Invitrogen Corporation), modified media thereof, mixed media thereof and the like. RPMI1640 medium, DMEM medium, F12 medium, IMDM, EX-CELL (registered trademark) 302 medium, hybridoma SFM medium (manufactured by Invitrogen Corporation) or the like is preferable.

Examples of the serum-containing medium in the invention include media obtained by adding serum of a mammal such as cow and horse, serum of a bird such as chicken, serum of fish such as yellowtail, one or more kinds of serum in any of the serum fractions above or a serum fraction to a minimal essential medium.

The serum-free medium in the invention is a medium obtained by adding a nutritional factor, a physiologically active substance or the like which is a substitute for serum, to a minimal essential medium.

The substance which is added as a substitute for the animal-derived component to the medium containing no animal-derived component in the invention is a physiologically active substance produced by a gene recombination method, a hydrolysate, a lipid containing no animal-derived material or the like.

Examples of the protein-free medium in the invention include ADPF medium (Animal derived protein free medium, manufactured by HyClone), CD-Hybridoma medium (manufactured by Invitrogen Corporation), CD-CHO medium (manufactured by Invitrogen Corporation), IS-CD-CHO medium (manufactured by Irvine Scientific), EX-CELL (registered trademark) CD-CHO medium (manufactured by SAFC Biosciences, Inc.) and the like.

Examples of the medium in the invention include a medium for culturing microorganisms, a medium for culturing insect cells, a medium for culturing yeast cells, a medium for culturing plant cells, a medium for culturing animal cells and the like, and a medium for culturing animal cells is preferably used.

As the medium for cell culture in the invention, any medium can be used as long as the medium is suitable for cell culture. A medium for culturing animal cells is preferable, and a medium for culturing Chinese hamster ovary (CHO) cells is further preferable.

The medium in the invention is not particularly restricted, and examples include a medium for expansion culture, a basal (starting) medium, a feed medium, a medium for reflux and the like.

As the powder medium in the invention, any medium can be used as long as the medium exists in a powder form. The powder medium in the invention also includes a medium which exists in a granular form.

The method for producing the powder medium in the invention is not particularly limited, and preferable methods include a production method by a mixing process of dry components using a disk mill, a ball mill, a pin mill or the like, a production method by freeze drying a liquid medium which is produced in advance and the like.

The method for producing the powder medium which exists in a granular form in the invention is not particularly limited and is Advanced Granulation Technology (registered trademark) or the like. The method may include a step of spraying and drying a solution obtained by further dissolving at least one material selected from the group consisting of a natural thickening agent, a synthetic thickening agent, a saccharide and an oil or a fat in addition to finely crushed components.

The liquid medium in the invention is not particularly limited and is preferably a medium in which cells or the like can be cultured. The liquid medium in the invention is prepared by dissolving the powder medium in a solvent.

As the solvent used for preparing the liquid medium, any solvent can be used as long as the powder medium dissolves, and examples include water, an aqueous solution containing a nutritional factor, a buffer component and/or the like and the like. A liquid medium can also be further used as a solvent.

The amount of the solvent used for preparing the liquid medium is not particularly limited as long as the amount is suitable for preparing the liquid medium in a desired amount required for producing the target polypeptide, and the amount is adjusted in the range of 10 L to 10000 L depending on the desired culture scale or the size of the culture vessel.

The nutritional factor in the invention includes a carbon source such as a saccharide and a nitrogen source such as an amino acid and in particular includes a saccharide, an amino acid, a vitamin, a lipid, a nucleic acid, a physiologically active substance, a fatty acid, an organic acid, a protein, a hydrolysate, a metal, a salt thereof or the like. The compounds may form a salt such as a hydrochloride, a sulfate, a nitrate, a sodium salt, a potassium salt and an ammonium salt and/or a solvate such as a hydrate.

The saccharide in the invention may be any of monosaccharides, oligosaccharides and polysaccharides and is not particularly limited. The saccharide also includes a sugar derivative such as deoxy sugar, an uronic acid, an amino sugar and a sugar alcohol. Examples include glucose, mannose, galactose, fructose, ribose, arabinose, ribulose, erythrose, erythrulose, glyceraldehyde, dihydroxyacetone, sedoheptulose, maltose, lactose, sucrose and the like, and one kind or a combination of two or more kinds is used.

The amino acid in the invention is not particularly limited. Examples include L-alanine (Ala), L-arginine (Arg), L-asparagine (Asn), L-aspartic acid (Asp), L-cysteine (Cys), L-glutamic acid (Glu), L-glutamine (Gln), glycine (Gly), L-histidine (His), L-isoleucine (Ile), L-leucine (Leu), L-lysine (Lys), L-methionine (Met), L-phenylalanine (Phe), L-proline (Pro), L-serine (Ser), L-threonine (Thr), L-tryptophan (Trp), L-valine (Val) and the like, and one kind or a combination of two or more kinds is used. A multimer such as cystine may also be used. The amino acid may be added in the form of a peptide or a multimer thereof, and examples include L-alanyl-L-glutamine, L-alanyl-L-cysteine, glutathione and the like.

The vitamin in the invention is not particularly limited. Examples include d-biotin, D-pantothenic acid, choline, folic acid, myo-inositol, niacinamide, pyridoxal, riboflavin, thiamine, cyanocobalamin, DL-α-tocopherol and the like, and one kind or a combination of two or more kinds is used.

The lipid in the invention is not particularly limited, and examples include cholesterol, linoleic acid, linolenic acid and the like.

The physiologically active substance in the invention is not particularly limited, and examples include insulin, transferrin, serum albumin, a serum fraction containing a growth factor and the like. The physiologically active substances also include physiologically active substances produced using a gene recombination technique or a chemical synthesis technique.

The hydrolysate in the invention is not particularly limited, and examples include hydrolysates of extracts or the like of soybean, wheat, rice, pea, cottonseed, fish or yeast, extracts and the like. Specific examples include SOY HYDROLYSATE UF (manufactured by SAFC Bioscience, catalogue number: 91052-1K3986 or 91052-5K3986) and the like.

The metal in the invention is not particularly limited. Examples include lithium, sodium, potassium, magnesium, calcium, iron, manganese, zinc, molybdenum, vanadium, copper, cadmium, rubidium, cobalt, zirconium, germanium, nickel, tin, chromium, silicon and the like, and one kind or a combination of two or more kinds is used.

The buffer component in the invention is not particularly limited, and examples include sodium hydrogen carbonate, disodium hydrogen phosphate, sodium dihydrogen phosphate, potassium hydrogen carbonate, dipotassium hydrogen phosphate, potassium dihydrogen phosphate, sodium acetate, acetic acid, sodium citrate, citric acid and the like.

The components of the powder medium or the liquid medium in the invention preferably include copper, cysteine, cystine and/or a derivative thereof.

The copper or the derivative thereof in the invention is not particularly limited and is a simple substance, an oxide, or a salt of copper, or a hydrate thereof. Examples of the oxide of copper include copper(I) oxide and copper(II) oxide. Examples of the salt of copper include copper(I) cyanide, copper(II) sulfate pentahydrate, copper(II) chloride dihydrate, copper(I) chloride, copper(II) nitrate trihydrate and the like, and copper(II) sulfate pentahydrate, copper(II) chloride dihydrate, copper(I) chloride or copper(II) nitrate trihydrate is preferable.

The copper or the derivative thereof is added in such a manner that the concentration in the prepared liquid medium becomes 1 ng/mL to 1 µg/mL, preferably 1 to 100 ng/mL, more preferably 5 ng/mL to 50 ng/mL.

The cysteine or the derivative thereof in the invention is not particularly limited and is L-cysteine, cysteine hydrochloride, cysteine hydrochloride monohydrate, acetylcysteine, glutathione or the like.

The cystine or the derivative thereof in the invention is not particularly limited and is L-cystine, cystine dihydrochloride, L-cystine disodium salt or the like.

The cysteine, the cystine or the derivative thereof is added in such a manner that the concentration in the prepared liquid medium becomes, for example, 1 µg/mL to 10 mg/mL.

The cysteine, the cystine or the derivative thereof may be dissolved in the solvent after adding to a powder medium containing the other components, or the cysteine, the cystine or the derivative thereof may be added to the solvent separately from a powder medium containing the other components. When the cysteine, the cystine or the derivative thereof is added to the solvent separately from a powder medium containing the other components, the cysteine, the cystine or the derivative thereof may be added before or after the powder medium containing the other components.

In the preparation of the liquid medium of the invention, oxygen may be supplied by sending a gas containing oxygen from the upper surface from the space in the upper surface of the container used for dissolving the powder medium and bringing the gas into contact with the solvent or the prepared liquid medium into contact, or oxygen may be supplied by sparging a gas containing oxygen into the solvent or into the prepared liquid medium.

The gas containing oxygen is not particularly limited as long as the gas contains oxygen, and examples include pure oxygen, a mixture of oxygen and another gas, air and the like. When the gas containing oxygen is a mixture of oxygen and another gas, the other gas is nitrogen, argon, helium, carbon dioxide or the like. One or more gases thereof and oxygen are prepared at any mixing ratio and supplied.

During the preparation of the liquid medium of the invention, the concentration or the amount of the supplied oxygen is not particularly limited as long as the medium components such as copper dissolve sufficiently and do not precipitate with the amount, and the concentration or the amount is preferably adjusted in such a manner that the solvent or the prepared liquid medium contains oxygen in an amount of 20% or more, 30% or more, 40% or more, 50% or more, 60% or more, 70% or more, 80% or more, 85% or more, 90% or more, 91% or more, 92% or more, 93% or more, 94% or more, 95% or more, 96% or more, 97% or more, 98% or more, 99% or more or 100% of the saturated dissolved oxygen. The amount is maintained at more preferably 20% or more, further preferably 40% or more of the saturated dissolved oxygen.

The method for maintaining the amount of oxygen contained in the solvent or the prepared liquid medium is a method for continuously or intermittently supplying a gas containing oxygen.

During the preparation of the liquid medium of the invention, the solvent or the prepared liquid medium is left still or stirred in the presence of oxygen. The leaving or stirring is conducted for a period with which the powder medium such as copper dissolves completely and with which the concentration can be maintained stably. Specifically, the period is in the range of 10 minutes to 10 days, preferably 30 minutes to 24 hours, more preferably 30 minutes to 6 hours.

By stirring, the powder medium and the oxygen can be dissolved in the solvent. The stirring is conducted by rotating a stirring blade, for example, at a speed of 1 to 200 rpm. Moreover, also through spontaneous mixing by sending a gas containing oxygen from the bottom surface of the solvent or the prepared liquid medium, the powder medium and the oxygen can be dissolved.

The amount of the supplied oxygen during the preparation of the liquid medium of the invention is, for example, 1 to 500 mL/minute, preferably 10 to 100 mL/minute per 1 liter of the solvent or the prepared liquid medium. The concentration or the amount of the oxygen supplied to the solvent or the prepared liquid medium can be easily controlled by measuring the dissolved oxygen concentration contained in the solvent or the prepared liquid medium by an optical or polarographic method or the like before or during the preparation of the liquid medium. By confirming that the oxygen concentration is appropriately controlled by measuring the dissolved oxygen concentration, the dissolution of the powder medium such as copper can be decided.

During the preparation of the liquid medium of the invention, the powder medium may be added to the solvent at once or added in several divided portions. Moreover, the powder may be introduced and dissolved in a container for medium preparation in which the solvent has already been put, or the solvent may be added after first introducing the powder into a container for medium preparation.

The application of the prepared liquid medium is not particularly limited, and the liquid medium can be used for cell culture after further filtrating using a membrane filter. That is, the method for preparing a liquid medium of the invention may further include a membrane filtration step after the step of dissolving the powder medium in the solvent in the presence of oxygen.

The membrane filtration method is not particularly limited as long as the liquid medium to be treated permeates a porous membrane by pressure and as long as undesirable substances such as microorganisms, components, particles and contaminants in the solution are removed by the method. Microfiltration, ultrafiltration, dialysis, electrodialysis or reverse osmosis is preferable, and microfiltration, ultrafiltration or dialysis is further preferable. Microfiltration is particularly preferable.

The membrane in the invention is not particularly limited and is preferably a microfiltration membrane, an ultrafiltration membrane, a dialysis membrane, an electrodialysis membrane or a reverse osmosis membrane. A microfiltration membrane, an ultrafiltration membrane or a dialysis membrane is further preferable. A microfiltration membrane is particularly preferable.

The pore size of the membrane filter used for the membrane filtration is not particularly limited as long as the liquid medium can be sterilized, and the pore size is preferably 1 nm to 100 µm, further preferably 5 nm to 10 µm, more preferably 10 nm to 1 µm. The pore size is particularly preferably 0.1 µm to 0.5 µm.

The material of the membrane used for the invention is not particularly limited, and examples include cellulose acetate, aromatic polyamides, polyacrylonitrile, polyvinyl chloride, polyvinyl chloride-polyacrylonitrile copolymer, polysulfone, polyethersulfone (PES), polyvinylidene fluoride (PVDF), ceramics, polyvinyl alcohol, polyvinylidene difluoride, mixed acetate-nitrate esters of cellulose, polytetrafluoroethylene, alumina, styrene-divinylbenzene copolymer, tetrafluoroethylene, derivatives thereof and the like. Polyethersulfone, polyvinylidene fluoride or the like is preferable.

Specific examples of the membrane filter using polyethersulfone or a derivative thereof include Millipore Express (registered trademark) PLUS Membrane Filters (pore size: 0.22 µm or 0.45 µm) (manufactured by Millipore), Millipore Express SHC cartridge filter (manufactured by Millipore), Millipore Express SHR cartridge filter (manufactured by Millipore), Supor EBV (manufactured by Pall Corporation), Supor EKV (manufactured by Pall Corporation, catalog number: AB3EKV7PH4), Supor Life 200 (manufactured by Pall Corporation), Sartopore (registered trademark) 2 (membrane structure: double-layer membrane, pore size: 0.2+0.1, 0.45+0.2 or 0.8+0.45 µm) (manufactured by sartorius stedim biotech), Sartopore (registered trademark) 2 XLG (membrane structure: double-layer membrane, pore size: 0.8+0.2 µm) (manufactured by sartorius stedim biotech), Sartopore (registered trademark) 2 XLI (membrane structure: double-layer membrane, pore size: 0.35+0.2 µm) (manufactured by sartorius stedim biotech), Sartopore 2 high flow (manufactured by sartorius stedim biotech), PES membrane cartridge filters TCS (pore size: 0.20 or 0.45 µm) (manufactured by ADVANTEC) and the like.

Specific examples of the membrane filter using polyvinylidene fluoride or a derivative thereof include Durapre (registered trademark) Membrane Filters (pore size: 0.10, 0.22, 0.45, 0.65 or 5.0 mm) (manufactured by Millipore), Durapore II Hydrophilic Filter Cartridge GV (manufactured by Millipore), Durapore II Hydrophilic Filter Cartridge VV (manufactured by Millipore), Fluorodyne (registered trademark) II-DFLP (manufactured by Pall Corporation), Fluorodyne II-DBLP (manufactured by Pall Corporation), Fluorodyne II-DJLP (manufactured by Pall Corporation), Ultipor VF-DV20 (manufactured by Pall Corporation), Ultipor VF-DV50 (manufactured by Pall Corporation) and the like.

Specific examples of the membrane filter using a combination of polyethersulfone or a derivative thereof and polyvinylidene fluoride or a derivative thereof include Fluorodyne (registered trademark) EX Grade EDF Membrane Filter Cartridge (manufactured by Pall Corporation, catalog number: AB3UEDF7PH4) and the like.

Examples of the membrane filter using a membrane material other than polyethersulfone or polyvinylidene fluoride include Omnipore (registered trademark) Membrane Filters (pore size: 0.1, 0.2, 0.45, 1.0, 5.0 or 10 µm) (manufactured by Millipore), MF-Millipore (registered trademark) Membrane Filters (pore size: 0.025, 0.05, 0.1, 0.22, 0.3, 0.45, 0.65, 0.8, 1.2, 3, 5 or 8 µm) (manufactured by Millipore), Nylon Membrane Filters (pore size: 0.20 or 5.0 µm) (manufactured by Millipore), Ultipor N66 (pore size: 0.2 or 0.45 µm) (manufactured by Pall Corporation), Posidyne (registered trademark) (pore size: 0.10, 0.20, 0.3 or 0.45 µm) (manufactured by Pall Corporation), Varafine VFSP (pore size: 0.2 or 0.45 µm) (manufactured by Pall Corporation), Varafine VFSE (pore size: 0.02, 0.1 or 0.2 µm) (manufactured by Pall Corporation), Varafine VFSG (pore size: 0.02, 0.1 or 0.2 µm) (manufactured by Pall Corporation), Sartolon (manufactured by sartorius stedim biotech), acetate membrane cartridge filters TCR (pore size: 0.20, 0.45 or 8.0 µm) (manufactured by ADVANTEC), Yumicron cartridge filters (pore size: 0.2, 0.4, 0.6, 0.9 or 2.5 µm) (manufactured by Yuasa Membrane Systems Co., Ltd.) and the like.

The membrane filter used for the invention may have a membrane filter structure composed of one piece, for example like Millex filter unit (manufactured by Millipore, catalog number: SLGV033RS), or may be a membrane having a structure composed of two or more layers or a combination of two or more pieces of membrane prepared by attaching one or more pieces of prefilter like 0.5/0.2 µm Express SHC Disk W/Typar (manufactured by Millipore, catalog number: HGEP02550).

The liquid medium prepared in the invention is characterized in that the concentration of a component in the liquid medium does not substantially change between before and after the membrane filtration. That the concentration of a component in the liquid medium does not substantially change between before and after the membrane filtration specifically means that, for example when the component is copper, the ratio of the copper concentration in the liquid medium after the membrane filtration to the copper concentration in the liquid medium before the membrane filtration is preferably 0.7 to 1.3, more preferably 0.8 to 1.2, further and most preferably 0.9 to 1.1.

The invention also relates to a method for culturing cells using the prepared liquid medium.

The cells in the invention may be either eukaryotic cells or prokaryotic cells, and examples include cells derived from mammals, birds, reptiles, amphibians, fish, insects, plants or the like, microorganisms such as bacteria, colon bacilli and hay bacilli, cells derived from microorganisms such as bacteria, colon bacilli and hay bacilli, yeasts and cells derived from yeasts and the like. The cells are preferably cells of an animal belonging to mammals, more preferably animal cells derived from a primate such as human and monkey or animal cells derived from a rodent such as mouse, rat and hamster, most preferably CHO cells derived from Chinese hamster ovary tissues.

The cells belonging to mammals in the invention are preferably myeloma cells, ovarian cells, kidney cells, blood cells, uterus cell connective tissue cells, mammary gland cells, embryonic retinoblasts, cells derived from the cells or the like, more preferably cells selected from myeloma cells, cells derived from myeloma cells, ovarian cells and cells derived from ovarian cells.

Examples include a human cell line HL-60 (ATCC No.CCL-240), HT-1080 (ATCC No.CCL-121), HeLa (ATCC No.CCL-2), 293 (ECACC No.85120602), Namalwa (ATCC CRL-1432), Namalwa KJM-1 [Cytotechnology, 1, 151 (1988)], NM-F9 (DSM ACC2605, WO2005/017130) or PER.C6 (ECACC No.96022940, U.S. patent No. 6855544 specification), monkey cell lines VERO (ATCC No.CCL-1651) and COS-7 (ATCC No.CRL-1651), a mouse cell line C127I (ATCC No.CRL-1616), Sp2/0-Ag14 (ATCC No.CRL-1581) or NIH3T3 (ATCC No.CRL-1658) NS0 (ATCC No.CRL-1827), a rat cell line Y3 Ag1.2.3. (ATCC No.CRL-1631), YO (ECACC No.85110501) or YB2/0 (ATCC No.CRL-1662), a hamster cell line CHO cells or BHK21 (ATCC No.CRL-10), canine cells MDCK (ATCC No.CCL-34) and the like.

Examples of the cells belonging to birds include a chicken cell line SL-29 (ATCC No.CRL-29) and the like. Examples of the cells belonging to fish include a zebrafish cell line ZF4 (ATCC No.CRL-2050) and the like. Examples of the cells belonging to insects include a moth (*Spodoptera frugiperda*) cell line Sf9 (ATCC No.CRL-1711) and the like. Examples of the primary culture cells used for vaccine production include primary monkey kidney cells, primary rabbit kidney cells, primary chicken embryo cells, primary quail embryo cells and the like.

The CHO cells in the invention include any cells as long as the cells are cells of a line established from ovary tissues of Chinese hamster *(Cricetulus griseus).* Specific examples thereof include CHO cells described in documents such as Journal of Experimental Medicine, 108, 945 (1958), Proc. Natl. Acad. Sci. USA, 60, 1275 (1968), Genetics, 55, 513 (1968), Chromosoma, 41, 129 (1973), Methods in Cell Science, 18, 115 (1996), Radiation Research, 148, 260 (1997), Proc. Natl. Acad. Sci. USA, 77, 4216 (1980), Proc. Natl. Acad. Sci. 60, 1275 (1968), Cell, 6, 121 (1975) and Molecular Cell Genetics, Appendix I,II, 883-900. Specific examples also include CHO-K1 line (ATCC No.CCL-61), DUXB11 line (ATCC CRL-9096), Pro-5 line (ATCC CRL-1781) and CHO/dhfr-(ATCC No.CRL-9096), which are registered in the ATCC (The American Type Culture Collection), commercial CHO-S line (Lifetechnologies Cat#11619), CHO/DG44 [Proc. Natl. Acad. Sci. USA, 77, 4216 (1980)], sublines obtained by conditioning the lines in various media and the like.

The cells in the invention are not particularly limited as to whether the cells have the capability of producing a substance or not, and examples include stem cells such as iPS cells obtained by introducing several genes to somatic cells, spermatids or egg cells taken from mammalian donors including a human and cells which produce a substance as well as fused cells which have become producing a substance and the like. Cells which produce a substance, fused cells which have become producing a substance and the like are particularly preferable. Animal cells which produce a substance, animal-derived fused cells which have become producing a substance and the like are further preferable, and, when the desired substance is an antibody, examples include a hybridoma which is a fused cell of an antibody-producing cell such as B cell and a myeloma cell and the like. Moreover, cells which have become producing a substance through mutation treatment, cells which have been subjected to mutation treatment to increase the expression level of a substance and the like are also included in the cells of the invention.

Examples of the cells which have become producing a substance through mutation treatment in the invention include cells obtained by introducing a mutation to a modification enzyme or the like so that the cells can produce a desired substance and the like. When the desired substance is a glycoprotein, examples include cells obtained by introducing mutations to various glycosylation enzymes to change the structures of the sugar chains in the glycoprotein and the like.

The cells which produce a substance in the invention may be any cells as long as the cells can produce a desired substance, and examples also include cells which have been transformed with a recombinant vector containing a gene that involves in the production of the substance. The transformed cells can be obtained, for example, by introducing a recombinant vector containing DNA that involves in the production of the substance and a promoter into host cells.

Examples of the gene that involves in the production of a substance in the invention include DNA which encodes a substance such as a peptide, a polypeptide, a protein, a glycoprotein and an antibody, DNA which encodes an enzyme or a protein that involves in the biosynthesis of a substance and the like.

As the promoter in the invention, any promoter can be used as long as the promoter functions in the cells, and examples include a promoter of the cytomegalovirus (CMV) immediate-early (IE) gene, the early promoter of SV40, a retroviral promoter, a metallothionein promoter, a heat shock promoter, SRα promoter and the like. Moreover, an enhancer of the IE gene of human CMV or the like may be used with the promoter.

The recombinant vector in the invention can be prepared using a desired vector. As the vector, any vector can be used as long as the vector functions in the cells, and examples include pcDNAI, pcDM8 (manufactured by Funakoshi Co., Ltd.), pAGE107 [JP-A-3-22979, Cytotechnology, 3, 133 (1990)], pAS3-3 (JP-A-2-227075), pcDM8 [Nature, 329, 840 (1987)], pcDNAI/Amp (manufactured by Invitrogen Corporation), pREP4 (manufactured by Invitrogen Corporation), pAGE103 [J. Biochem., 101, 1307 (1987)], pAGE210 and the like.

As the method for introducing the recombinant vector into the host cells in the invention, any method can be used as long as the method is for introducing DNA into cells, and examples include the electroporation [Cytotechnology, 3, 133 (1990)], the calcium phosphate method (JP-A-2-227075), the lipofection [Proc. Natl. Acad. Sci. USA, 84, 7413 (1987), Virology, 52, 456 (1973)] and the like.

Examples of the transformed cells in the invention include anti-GD3 human chimeric antibody-producing transformed cells 7-9-51 (FERM BP-6691), anti-CCR4 chimeric antibody-producing transformed cells KM2760 (FERM BP-7054), anti-CCR4 humanized antibody-producing transformed cells KM8759 (FERM BP-8129) and KM8760 (FERM BP-8130), 709 LCA-500D (FERM BP-8239), anti-IL-5 receptor α chain chimeric antibody-producing transformed cells KM7399 (FERM BP-5649), anti-IL-5 receptor α chain human CDR-grafted antibody-producing transformed cells KM8399 (FERM BP-5648) and KM9399 (FERM BP-5647), anti-GM2 human CDR-grafted antibody-producing transformed cells KM8966 (FERM BP-5105), KM8967 (FERM BP-5106), KM8969 (FERM BP-5527), KM8970 (FERM BP-5528), an anti-CD20 antibody-producing transformant line Ms704-CD20 (FERM BP-10092), antithrombin III-producing transformed cells Ms705-pKAN-ATIII (FERM BP-8472) and the like.

The method for culturing cells in the invention is a method suitable for the cells used, such as batch culture, repeated batch culture, rolling seed culture, fed-batch culture and perfusion culture, and fed-batch culture is preferably used. The cells are cultured generally under the conditions of pH 6 to 8 at 30 to 40°C or the like, for example, for 3 to 20 days in the case of fed-batch culture and for 3 to 60 days in the case of perfusion culture. Depending on the need, an antibiotic such as streptomycin and penicillin may be added to the medium during the culture. For controlling the dissolved oxygen concentration, controlling the pH, controlling the temperature, stirring and the like, methods which are used for general cell culture can be used.

The culture volume of the culture method in the invention may be any culture volume such as a very low culture volume of generally 0.1 mL to 10 mL using a plate for cell culture, a low culture volume of generally 10 to 1000 mL using an Erlenmeyer flask or the like, a high culture volume of generally 1 to 20000 L using a culture bath such as a jar or the like which can be used for commercial production.

The invention also relates to a method for producing a polypeptide characterized by culturing cells that express a desired polypeptide in the prepared liquid medium, producing and accumulating the polypeptide in a culture and collecting the polypeptide from the culture or to a polypeptide produced using the production method.

The polypeptide in the invention may be any polypeptide as long as the polypeptide is composed of amino acids linked by peptide bonds, and the polypeptide is preferably a eukaryotic cell-derived polypeptide, further preferably an animal cell-derived polypeptide such as a mammalian cell-derived polypeptide. The polypeptide of the invention may also be, for example, an artificially modified polypeptide such as a fusion peptide obtained through fusion of a desired polypeptide and another polypeptide or a polypeptide composed of a partial fragment. An example is a polypeptide having physiological activity.

Examples of the polypeptide in the invention include a protein, a glycoprotein, an antibody, a polypeptide composed of a partial fragment thereof and the like.

Examples of the polypeptide of the invention also include a polypeptide which regulates the activity of an enzyme, a polypeptide which has the structure of an enzyme and the like. Specific examples of the polypeptide which regulates the activity of an enzyme include a polypeptide which functions as an agonist or an antagonist of a glycoprotein and the like. The agonist may be any agonist as long as the agonist is a polypeptide which has an activity of enhancing the activity of a glycoprotein, and the agonist is specifically a somatostatin derivative, somatropin, atrial natriuretic peptide, glucagon, insulin, an insulin-like growth factor, a gonadotropin or the like. The antagonist may be any antagonist as long as the antagonist is a polypeptide which has an activity of inhibiting the activity of a glycoprotein, and the antagonist is specifically pegvisomant or the like.

Examples of the protein or the glycoprotein in the invention include erythropoietin (EPO) [J. Biol. Chem., 252, 5558 (1977)], thrombopoietin (TPO) [Nature, 369 533 (1994)], tissue plasminogen activator, pro-urokinase, thrombomodulin, antithrombin III, protein C, protein S, blood coagulation factor VII, blood coagulation factor VIII, blood coagulation factor IX, blood coagulation factor X, blood coagulation factor XI, blood coagulation factor XII, prothrombin complex, fibrinogen, albumin, gonadotropin, thyroid-stimulating hormone, epidermal growth factor (EGF), hepatocyte growth factor (HGF), keratinocyte growth factor, activin, osteogenic factor, stem cell factor (SCF), granulocyte colony-stimulating factor (G-CSF) [J. Biol. Chem., 258, 9017 (1983)], macrophage colony-stimulating factor (M-CSF) [J. Exp. Med., 173, 269 (1992)], granulocyte-macrophage colony-stimulating factor (GM-CSF) [J. Biol. Chem., 252, 1998 (1977)], interferon α, interferon β, interferon γ, interleukin-2 (IL-2) [Science, 193, 1007 (1976)], interleukin 6, interleukin 10, interleukin 11, interleukin-12 (IL-12) [J. Leuc. Biol., 55, 280 (1994)], soluble interleukin 4 receptor, tumor necrosis factor α, DNaseI, galactosidase, α glucosidase, glucocerebrosidase, hemoglobin, transferrin, derivatives thereof, partial fragments thereof and the like.

The antibody in the invention may be any antibody as long as the antibody has the antigen-binding capacity, and examples include an antibody which recognizes a tumor-associated antigen, an antibody fragment thereof, an antibody which recognizes an allergy- or inflammation-associated antigen, an antibody fragment thereof, an antibody which recognizes a cardiovascular disease-associated antigen, an antibody fragment thereof, an antibody which recognizes an autoimmune disease-associated antigen, an antibody fragment thereof, an antibody which recognizes a viral or bacterial infection-associated antigen, an antibody fragment thereof and the like.

The tumor-associated antigens in the invention include CD1a, CD2, CD3, CD4, CD5, CD6, CD7, CD9, CD10, CD13, CD19, CD20, CD21, CD22, CD25, CD28, CD30, CD32, CD33, CD38, CD40, CD40 ligand (CD40L), CD44, CD45, CD46, CD47, CD52, CD54, CD55, CD56, CD59, CD63, CD64, CD66b, CD69, CD70, CD74, CD80, CD89, CD95, CD98, CD105, CD134, CD137, CD138, CD147, CD158, CD160, CD162, CD164, CD200, CD227, adrenomedullin, angiopoietin related protein 4 (ARP4), aurora, B7-H1, B7-DC, integrin, bone marrow stromal antigen 2 (BST2), CA125, CA19.9, carbonic anhydrase 9 (CA9), cadherin, cc-chemokine receptor (CCR) 4, CCR7, carcinoembryonic antigen (CEA), cysteine-rich fibroblast growth factor receptor-1 (CFR-1), c-Met, c-Myc, collagen, CTA, connective tissue growth factor (CTGF), CTLA-4, cytokeratin-18, DF3, E-catherin, epidermal growth facter receptor (EGFR), EGFRvIII, EGFR2 (HER2), EGFR3 (HER3), EGFR4 (HER4), endoglin, epithelial cell adhesion molecule (EpCAM), endothelial protein C receptor (EPCR), ephrin, ephrin receptor (Eph), EphA2, endotheliase-2 (ET2), FAM3D, fibroblast activating protein (FAP), Fc receptor homolog 1 (FcRH1), ferritin, fibroblast growth factor 8 (FGF8), FGF8 receptor, basic FGF (bFGF), bFGF receptor, FGF receptor (FGFR) 3, FGFR4, FLT1, FLT3, folate receptor, frizzled homologue 10 (FZD10), frizzled receptor 4 (FZD-4), G250, G-CSF receptor, gangliosides (GD2, GD3, GM2, GM3 or the like), globo H, gp75, gp88, GPR-9-6, heparanase I, hepatocyte growth factor (HGF), HGF receptor, HLA antigens (HLA-DR or the like), HM1.24, human milk fat globule (HMFG), hRS7, heat shock protein 90 (hsp90), idiotype epitope, insulin-like growth factor (IGF), IGF receptor (IGFR), interleukins (IL-6, IL-15 or the like), interleukin receptors (IL-6R, IL-15R or the like), integrin, immune receptor translocation associated-4 (IRTA-4), kallikrein 1, KDR, KIR2DL1, KIR2DL2/3, KS1/4, lamp-1, lamp-2, laminin-5, Lewis y, sialyl Lewis x, lymphotoxin-beta receptor (LTBR), LUNX, melanoma-associated chondroitin sulfate proteoglycan (MCSP), mesothelin, MICA, Mullerian inhibiting substance type II receptor (MISIIR), mucin, neural cell adhesion molecule (NCAM), Necl-5, Notchl, osteopontin, platelet-derived growth factor (PDGF), PDGF receptor, platelet factor-4 (PF-4), phosphatidylserine, Prostate Specific Antigen (PSA), prostate stem cell antigen (PSCA), prostate specific membrane antigen (PSMA), Parathyroid hormone related protein/peptide (PTHrP), receptor activator of NF-kappaB ligand (RANKL), receptor for hyaluronic acid mediated motility (RHAMM), ROBO1, SART3, semaphorin 4B (SEMA4B), secretory leukocyte protease inhibitor (SLPI), SM5-1, sphingosine-1-phosphate, tumor-associated glycoprotein-72 (TAG-72), transferrin receptor (TfR), TGF-beta, Thy-1, Tie-1, Tie2 receptor, T cell immunoglobulin domain and mucin domain 1 (TIM-1), human tissue factor (hTF), Tn antigen, tumor necrosis factor (TNF), Thomsen-Friedenreich antigen (TF antigen), TNF receptor, tumor necrosis factor-related apoptosis-inducing ligand (TRAIL), TRAIL receptors (DR4, DR5or the like), system ASC amino acid transporter 2 (ASCT2), trkC, TROP-2, TWEAK receptor Fn14, type IV collagenase, urokinase receptor, vascular endothelial growth factor (VEGF), VEGF receptors (VEGFR1, VEGFR2, VEGFR3 or the like), vimentin, VLA-4 and the like.

The antibody in the invention may be either a monoclonal antibody or a polyclonal antibody. The classes of the antibody include immunoglobulin G (IgG), immunoglobulin A (IgA), immunoglobulin E (IgE) and immunoglobulin M (IgM), and the class is preferably IgG. The subclass of IgG is IgG1, IgG2, IgG3 or IgG4.

The antibody in the invention includes a partial fragment containing a part of an antibody or the like, and examples include Fab (Fragment of antigen binding), Fab', F(ab')2, single chain antibodies (single chain Fv, scFv), disulfide stabilized antibodies (disulfide stabilized Fv, dsFv), fusion proteins containing the Fc region of an antibody and the like.

Examples of the antibody in the invention include an antibody secreted by hybridoma cells produced from spleen cells of an immunized animal obtained by immunizing the animal with an antigen and an antibody produced by a gene recombination technique. The antibody produced by a gene recombination technique can be obtained, for example by introducing an antibody expression vector obtained by inserting an antibody gene into a host cell. Specific examples include an antibody produced by a hybridoma, a human chimeric antibody, a humanized antibody, a human antibody and the like.

The human chimeric antibody in the invention is an antibody composed of an antibody heavy chain variable region (each also referred to as HV or VH below, where a heavy chain is a H chain, and a variable region is a V region) and an antibody light chain variable region (each also referred to as LV or VL below, where a light chain is a L chain) of an animal other than human, a heavy chain constant region (also referred to as CH below, where a constant region is a C region) of a human antibody and a light chain constant region (also referred to as CL below) of a human antibody. As the animal other than human, any animal, such as mouse, rat, hamster and rabbit, can be used as long as a hybridoma can be produced.

The human chimeric antibody can be obtained by obtaining cDNAs encoding VH and VL from a non-human animal cell-derived hybridoma which produces a monoclonal antibody, inserting the cDNAs into an expression vector for host cells having genes encoding human antibody CH and human antibody CL, thereby constructing a human chimeric antibody expression vector, introducing the expression vector into the host cells and expressing the antibody.

The CH of the human chimeric antibody may be any CH as long as the CH belongs to human immunoglobulin (referred to as hlg below). CH of the class hIgG is preferable, and any CH of a subclass belonging to the class hIgG such as hIgG1, hIgG2, hIgG3 and hIgG4 can be used. Moreover, the CL of the human chimeric antibody may be any CL as long as the CL belongs to hlg, and CL of the class κ or the class λ can be used.

Examples of the humanized antibody in the invention include a complementarity determining region (referred to as CDR below)-grafted antibody produced by grafting the amino acid sequences of the human CDRs of VH and VL of an antibody of an animal other than human to appropriate positions of VH and VL of a human antibody and the like.

The CDR-grafted antibody can be obtained by constructing cDNAs encoding the V regions in which the CDR sequences of VH and VL of an antibody of an animal other than human are grafted to the CDR sequences of VH and VL of any human antibody, inserting the cDNAs into an expression vector for host cells having genes encoding CH of a human antibody and CL of a human antibody, thereby constructing a CDR-grafted antibody expression vector, introducing the expression vector to host cells and expressing the antibody.

The CH of the CDR-grafted antibody may be any CH as long as the CH belongs to hlg. CH of the class hIgG is preferable, and any CH of a subclass belonging to the class hIgG such as hIgG1, hIgG2, hIgG3 and hIgG4 can be used. Moreover, the CL of the CDR-grafted antibody may be any CL as long as the CL belongs to hlg, and CL of the class κ or the class λ can be used.

The human antibody in the invention can be obtained, for example, by culturing and purifying lymphocytes which produce the human antibody and which are obtained by isolating human peripheral blood lymphocytes, infecting with EB virus or the like, immortalizing and cloning the lymphocytes.

The human antibody in the invention can also be obtained by inserting the antibody gene prepared from human B cells into a phage gene, thereby obtaining a human antibody phage library in which antibody fragments such as Fab and scFv are expressed on the phage surface, collecting a phage which expresses an antibody fragment having the antigen-binding capacity using the avidity to the immobilized antigen as an index and converting the antibody fragment into a human antibody molecule including two complete H chains and two complete L chains.

The human antibody can be obtained also from a human antibody-producing hybridoma which is obtained from a human antibody-producing transgenic animal in which the human antibody gene has been incorporated into the cells by a hybridoma production method or the like which is generally used for mammals other than human. Specifically, a human antibody-producing hybridoma can be obtained by introducing the human antibody gene into mouse ES cells, grafting the ES cells into a mouse early embryo and then developing the embryo to obtain a human antibody-producing transgenic mouse.

The human antibody can be obtained by obtaining cDNAs encoding VL and VH from the human antibody-producing hybridoma, inserting the cDNAs into an expression vector for host cells having DNA encoding CL and CH of the human antibody in which one or more amino acid residues of the wild type (described as WT below) have been replaced with Cys residues appropriately by the above method or the like, introducing the vector into host cells and expressing the antibody.

Alternatively, the human antibody can be obtained also by obtaining cDNAs encoding VL and VH from the human antibody-producing hybridoma, inserting the cDNAs into an expression vector for host cells having DNA encoding CL and CH of the human antibody, replacing one or more amino acid residues of the WT with Cys residues appropriately by the above method or the like to construct a human antibody expression vector, introducing the vector into animal cells and expressing the antibody.

The CH of the WT used for the human antibody may be any CH as long as the CH belongs to hlg. CH of the class hIgG is preferably used, and any CH of a subclass belonging to the class hIgG such as hIgG1, hIgG2, hIgG3 and hIgG4 can be used. Moreover, the CL of the human antibody may be any CL as long as the CL belongs to hlg, and CL of the class κ or the class λ can be used.

Examples of the antibody in the invention include the following antibodies, but the antibody is not particularly limited to these examples.

Examples of the antibody which recognizes a tumor-associated antigen include anti-GD2 antibody [Anticancer Res., 13, 331 (1993)], anti-GD3 antibody [Cancer Immunol. Immunother., 36, 260 (1993)], anti-GM2 antibody [Cancer Res., 54, 1511 (1994)], anti-HER2 antibody [Proc. Natl. Acad. Sci. USA, 89, 4285 (1992), U.S. patent No. 5725856 specification], anti-CD52 antibody [Proc. Natl. Acad. Sci. USA, 89, 4285 (1992)], anti-MAGE antibody [British J. Cancer, 83, 493 (2000)], anti-HM1.24 antibody [Molecular Immunol., 36, 387 (1999)], anti-parathyroid hormone-related protein (PTHrP) antibody [Cancer, 88, 2909 (2000)], anti-bFGF antibody, anti-FGF-8 antibody [Proc. Natl. Acad. Sci. USA, 86, 9911 (1989)], anti-bFGFR antibody, anti-FGF-8R antibody [J. Biol. Chem., 265, 16455 (1990)], anti-IGF antibody [J. Neurosci. Res., 40, 647 (1995)], anti-IGF-IR antibody [J. Neurosci. Res., 40, 647 (1995)], anti-PSMA antibody [J. Urology, 160, 2396 (1998)], anti-VEGF antibody [Cancer Res., 57, 4593 (1997)], anti-VEGFR antibody [Oncogene, 19, 2138 (2000), WO1996/30046], anti-CD20 antibody [Curr. Opin. Oncol., 10, 548 (1998), U.S. patent No. 5736137 specification], anti-CDIO antibody, anti-EGFR antibody (WO1996/402010), anti-Apo-2R antibody (WO1998/51793), anti-ASCT2 antibody (WO2010/008075), anti-CEA antibody [Cancer Res., 55 (23 suppl): 5935s-5945s, (1995)], anti-CD38 antibody, anti-CD33 antibody, anti-CD22 antibody, anti-EpCAM antibody, anti-A33 antibody and the like.

Examples of the antibody which recognizes an allergy- or inflammation-associated antigen include anti-interleukin 6 antibody [Immunol. Rev., 127, 5 (1992)], anti-interleukin 6 receptor antibody [Molecular Immunol., 31, 371 (1994)], anti-interleukin 5 antibody [Immunol. Rev., 127, 5(1992)], anti-interleukin 5 receptor antibody, anti-interleukin 4 antibody [Cytokine, 3, 562 (1991)], anti-interleukin 4 receptor antibody [J. Immunol. Methods, 217, 41 (1998)], anti-tumor necrosis factor antibody [Hybridoma, 13, 183 (1994)], anti-tumor necrosis factor receptor antibody [Molecular Pharmacol., 58, 237 (2000)], anti-CCR4 antibody [Nature, 400, 776, (1999)], anti-chemokine antibody (Peri et al., J. Immunol. Meth. 174, 249, 1994), anti-chemokine receptor antibody [J. Exp. Med., 186, 1373 (1997)] and the like.

Examples of the antibody which recognizes a cardiovascular disease-associated antigen include anti-GPIIb/IIIa antibody [J. Immunol., 152, 2968 (1994)], anti-platelet-derived growth factor antibody [Science, 253, 1129 (1991)], anti-platelet-derived growth factor receptor antibody [J. Biol. Chem., 272, 17400 (1997)], anti-blood coagulation factor antibody [Circulation, 101, 1158 (2000)], anti-IgE antibody, anti-αVβ3 antibody, α4β7 antibody and the like.

Examples of the antibody which recognizes a viral or bacterial infection-associated antigen include anti-gp120 antibody [Structure, 8, 385 (2000)], anti-CD4 antibody [J. Rheumatology, 25, 2065 (1998)], anti-CCR5 antibody, anti-verotoxin antibody [J. Clin. Microbiol., 37, 396 (1999)] and the like.

As the method for producing a polypeptide of the invention, for example, a direct expression method for intracellularly producing the polypeptide, a method for extracellularly secreting and producing the polypeptide (Molecular Cloning, second edition) or the like can be used.

The polypeptide of the invention can be actively secreted outside the host cells using the method of Paulson et al. [J. Biol. Chem., 264, 17619 (1989)], the method of Lowe et al. [Proc. Natl. Acad. Sci. USA, 86, 8227 (1989), Genes Develop., 4, 1288 (1990)] or a method described in JP-A-5-336963, WO1994/23021 or the like. That is, a desired polypeptide can be actively secreted outside the host cells by expressing the desired polypeptide in the form of having a signal peptide at the N terminal using a gene recombination method.

Moreover, the production amount of the polypeptide can be increased using the gene amplification system using dihydrofolate reductase gene or the like described in JP-A-2-227075.

The polypeptide produced by the method of the invention can be isolated and purified, for example, using a general method for isolating and purifying a polypeptide or the like.

When the desired polypeptide is intracellularly expressed in a dissolved state, the cells are collected by centrifugation after completing culture and suspended in an aqueous buffer solution, followed by crushing of the cells with an ultrasonic crusher, a French press, a Manton Gaulin homogenizer, a Dyno mill or the like, and a cell-free extract is thus obtained. A partially purified sample or a purified sample can be obtained from a supernatant obtained by centrifugating the cell-free extract using a general method for isolating and purifying a peptide or a protein, namely, a solvent extraction method, a salting-out method using ammonium sulfate or the like, a desalting method, a precipitation method using an organic solvent, anion-exchange chromatography using a resin such as Diethylaminoethyl-Sepharose and DIAION HPA-75 (manufactured by Mitsubishi Kasei Corporation), cation-exchange chromatography using a resin such as S-Sepharose FF (manufactured by Pharmacia), hydrophobic interaction chromatography using a resin such as Butyl Sepharose and Phenyl Sepharose, a gel filtration method using a molecular sieve, affinity chromatography using a resin containing protein A, protein G or the like, a chromatofocusing method, electrophoresis such as isoelectric focusing electrophoresis or the like, alone or in combination.

When the desired polypeptide is extracellularly secreted, the polypeptide can be collected in a culture supernatant. That is, a partially purified sample or a purified sample can be obtained by treating the culture with the same method as that described above such as centrifugation, thereby obtaining a culture supernatant, and then obtaining from the culture supernatant using the same isolation and purification method as that described above.

The invention also relates to a method for controlling the generation of a variant derived from a polypeptide in which the prepared liquid medium is used in a step of culturing cells that express the polypeptide.

The variant derived from a polypeptide in the invention is not particularly limited, and examples include a modified product, a saccharified product, an oxide, a sugar chain-modified product such as a high-mannose product, a polymer, a decomposition product and the like which are derived from the desired polypeptide.

For example, it is known that amidated proline is generated by peptidylglycine α-amidation monooxygenase in the presence of copper in a liquid medium (K.A. Johnson et al., Anal Bochem 2007). Moreover, a high-mannose product, a saccharified product, an oxide, a polymer, a decomposition product and the like are also variants which can be controlled with the concentration of copper in the liquid medium [Tailoring Recombinant Protein Quality by Rational Media DesignBiotechnol. Prog. 31 (3), 615-629, 2015].

Using the prepared liquid medium of the invention, the generation amount of such a variant or the variant content can be controlled at any content, and, for example, the generation of a variant derived from a desired polypeptide which changes with the concentration of copper in the liquid medium can be controlled.

The amount of the variant derived from a polypeptide in the invention can be measured, for example, using a separation method using ion-exchange chromatography. For example, when an antibody is analyzed by cation-exchange column chromatography, in general, peaks which are eluted before the major peak contain acidic variants with a low pI value and called acidic peaks. The components eluted after the major peak contain basic variants with a high pI value and called basic peaks.

The invention is explained more specifically referring to Examples below, but the Examples are mere examples of the invention and do not limit the scope of the invention.

### Examples

### [Example 1] Influence of Presence or Absence of Oxygen During Medium Preparation on Copper Concentration

The influence of the oxygen concentration in a liquid medium containing a powder medium during the preparation of the medium on the copper concentration of the medium after the preparation and filtration was assessed.

The media were prepared by the following procedures.

First, pure water was poured into two preparation containers. Air was sent to one of the containers from the bottom surface, and the dissolved oxygen concentration was saturated. Nitrogen was sent to the other container from the bottom surface, and the dissolved oxygen was discharged from the system. To the containers, 6.645 g/L of powder medium A (manufactured by FUJIFILM Wako Pure Chemical Corporation) containing amino acids, metal salts, vitamins and the like, 0.510 g/L of L(+)-glutamine (manufactured by SIGMA), 1.250 g/L of SOY HYDROLYSATE UF (manufactured by SAFC Bioscience), 10.0 mL/L of PLURONIC F-68 10% Solution (manufactured by Thermo Fidher Scientific), 2.535 g/L of sodium chloride (manufactured by Tomita Pharmaceutical Co., Ltd.), 8.137 g/L of powder medium B (manufactured by Thermo Fidher Scientific), 0.125 mL/L of powder medium C (manufactured by Thermo Fidher Scientific) and 6.768 g/L of powder medium D (manufactured by Thermo Fidher Scientific) were added, and the mixtures were stirred for about 60 minutes. After further adding 2.380 g/L of sodium hydrogen carbonate (manufactured by Kanto Chemical Co., Inc.) and stirring for about 10 minutes, the volumes were increased to 2.5 L by adding pure water.

Next, the prepared liquid media were stirred continuously while air or nitrogen was sent, and samples were taken from the media during preparation after 0 minutes, after 60 minutes and after 180 minutes of stirring and filtered through a filter having a pore size of 0.1 µm. The copper concentrations of the media before the filtration and after the filtration were analyzed using ICP-MS (manufactured by Agilent Technologies).

As a result, as shown in Fig. 1(A), no change was found in the copper concentration of the medium prepared while sending air between before and after the filter filtration even after continuously stirring. The copper concentration after 180 minutes of stirring before the filtration was 11.5 ng/mL, while the copper concentration after the filtration was 10.9 ng/mL. On the other hand, as shown in Fig. 1(B), the copper concentration of the medium prepared while sending nitrogen to remove oxygen after 180 minutes of stirring before the filtration was 11.4 ng/mL, while the copper concentration after the filtration was 6.6 ng/mL, which showed that the concentration decreased to about a half.

From the above results, it was shown that the copper concentration of a medium after filter filtration decreases when the dissolved oxygen concentration is low during the preparation of the medium.

### [Example 2] Assessment of Amount of Supplied Oxygen for Dissolving Copper in Medium

The influence of the dissolved oxygen concentration in a liquid medium containing a powder medium during the preparation of the medium on the copper concentration of the medium after the preparation and filtration was assessed.

The media were prepared by the following procedures.

First, pure water was poured into four preparation containers. Air was sent to one of the containers from the bottom surface, and the dissolved oxygen concentration was saturated. A mixture gas of nitrogen and oxygen was sent to the remaining three containers, and the dissolved oxygen concentrations were controlled to 40%, 20% or 0%. While air or the mixture gas of nitrogen and oxygen was continuously sent to the preparation containers to keep the dissolved oxygen concentrations, 6.645 g/L of powder medium A (manufactured by FUJIFILM Wako Pure Chemical Corporation) containing amino acids, metal salts, vitamins and the like, 0.510 g/L of L(+)-glutamine (manufactured by SIGMA), 1.250 g/L of SOY HYDROLYSATE UF (manufactured by SAFC Bioscience), 10.0 mL/L of PLURONIC F-68 10% Solution (manufactured by Thermo Fidher Scientific), 2.535 g/L of sodium chloride (manufactured by Tomita Pharmaceutical Co., Ltd.), 8.137 g/L of powder medium B (manufactured by Thermo Fidher Scientific), 0.125 mL/L of powder medium C (manufactured by Thermo Fidher Scientific) and 6.768 g/L of powder medium D (manufactured by Thermo Fidher Scientific) were added, and the mixtures were stirred for about 60 minutes. After further adding 2.380 g/L of sodium hydrogen carbonate (manufactured by Kanto Chemical Co., Inc.) and stirring for about 10 minutes, the volumes were increased to 2.5 L by adding pure water.

Next, the prepared liquid media were stirred continuously while air or the mixture gas of nitrogen and oxygen was sent to keep the dissolved oxygen concentrations, and samples were taken from the media during preparation 240 minutes after introducing the raw materials and filtered through a filter having a pore size of 0.1 µm. The copper concentrations of the media before the filtration and after the filtration were analyzed using ICP-MS (manufactured by Agilent Technologies).

The results are shown in Fig. 2. While the copper concentration of the medium prepared under the conditions of the dissolved oxygen concentration in the prepared medium of 0% after the filtration was 1.4 ng/mL, the copper concentrations of the media prepared under the conditions of the dissolved oxygen concentrations of 20%, 40% and 100% (atmospheric saturation state) after the filtration were 8.5, 12.0 and 10.9 ng/mL, respectively.

From the above results, it was shown that the copper concentration in a medium after filter filtration decreases when the dissolved oxygen concentration becomes lower than 40% during the preparation of the medium.

### [Example 3] Relation Between Copper Concentration of Medium and Quality of Produced Antibody

The qualities of an antibody prepared using media in which the copper concentrations of the media after filtration were changed with the dissolved oxygen concentrations during the preparation of the media were checked, and it was shown that the variant content can be controlled in a concentration-dependent manner.

CHO cells producing an IgG-type monoclonal antibody were cultured using the four kinds of liquid medium prepared in Example 3, and the antibody was prepared. The medium preparation and the analysis were conducted by the following procedures.

CHO cells into which the antibody gene had been incorporated were seeded to the media prepared in Example 3 and cultured by a fed-batch process while additives such as amino acids and glucose were added. The target antibody was thus produced. After the completion of culture, the cells were removed. Then, the culture supernatants were collected, and the antibody was purified. Variants were detected from the purified antibody by high-performance liquid chromatography using a cation-exchange chromatography column. The correlations between the peaks containing basic variants (basic peaks), of the variants detected by cation-exchange chromatography, and the copper concentrations of the media were compared.

The results are shown in Fig. 3. When the media prepared with the dissolved oxygen concentrations kept at 100% (atmospheric saturation state), 40% and 20% (the copper concentrations of the media were 10.9, 12.0 and 8.5 ng/mL, respectively) were used, the percentages of the basic peaks were 13.0%, 13.5% and 12.4%, respectively, which had no large difference. On the other hand, when the medium prepared with the dissolved oxygen concentration kept at 0% (the copper concentration of the medium was 1.4 ng/mL) was used, the percentage of the basic peaks decreased to 10.0%.

From the above results, it was shown that the copper concentration of a medium after filtration changes as the dissolved oxygen concentration during the preparation of the medium changes, which results in a copper concentration-dependent decrease in the percentage of the basic peaks of the antibody produced by animal cells, and that the percentage of the basic peaks can be controlled depending on the copper concentration.

Although the invention has been explained in detail using specific embodiments, it is obvious to one skilled in the art that various changes and modifications can be made without departing from the intension and the scope of the invention. The present application is based on a U.S. provisional application filed on November 2, 2018 (US62/754793), which is incorporated by reference in its entirety.

### Industrial Applicability

The invention provided a preparation method in which the copper concentration of a liquid medium does not substantially change during membrane filtration when a powder medium containing copper and cysteine, cystine or a derivative thereof is dissolved in a solvent. A liquid medium prepared by the preparation method, a method for culturing cells using a liquid medium prepared by the preparation method, a method for producing a physiologically active substance using the culture method, a physiologically active substance produced using the production method, a membrane filtration method of a liquid medium prepared by the preparation method, a method in which copper is not removed by membrane filtration by controlling the amount of supplied dissolved oxygen or a method for producing a physiologically active substance by preparing and membrane filtrating a liquid medium and culturing cells using the liquid medium was provided.

## Claims

1. A method for preparing a liquid medium, comprising a step of dissolving a powder medium in a solvent in the presence of oxygen.

2. The method for preparing a liquid medium according to claim 1, wherein the powder medium comprises copper or a derivative thereof.

3. The method for preparing a liquid medium according to claim 2, wherein the copper or the derivative thereof is a simple substance, an oxide, or a salt of copper or a hydrate thereof.

4. The method for preparing a liquid medium according to claim 2 or 3, wherein the copper or the derivative thereof is copper(II) sulfate pentahydrate, copper(II) chloride dihydrate, copper(I) chloride, copper(II) nitrate trihydrate or a mixture thereof.

5. The method for preparing a liquid medium according to any one of claims 1 to 4, comprising a step of adding cysteine, cystine or a derivative thereof.

6. The method for preparing a liquid medium according to any one of claims 1 to 5, comprising at least one operation selected from the following (i) and (ii):
(i) an operation of sending a gas containing oxygen from the upper surface and bringing the gas into contact with the solvent; and
(ii) an operation of sparging the gas containing oxygen into the solvent.

7. The method for preparing a liquid medium according to any one of claims 1 to 6, comprising an operation of stirring the solvent.

8. The method for preparing a liquid medium according to any one of claims 1 to 7, further comprising a membrane filtration step after the step of dissolving the powder medium in the solvent in the presence of oxygen.

9. The method for preparing a liquid medium according to claim 8, wherein a copper concentration in the liquid medium does not substantially change between before and after the membrane filtration.

10. The method for preparing a liquid medium according to claim 8 or 9, wherein a pore size of a membrane filter used for the membrane filtration is 1 nm to 100 µm.

11. The method for preparing a liquid medium according to any one of claims 1 to 10, comprising a step of measuring a dissolved oxygen concentration in the solvent or the liquid medium.

12. The method for preparing a liquid medium according to any one of claims 1 to 11, comprising a step of measuring the dissolved oxygen concentration in the solvent or the liquid medium before or during the preparation of the liquid medium.

13. The method for preparing a liquid medium according to any one of claims 1 to 12, wherein the solvent contains oxygen in an amount of 20% or more, 30% or more, 40% or more, 50% or more, 60% or more, 70% or more, 80% or more, 85% or more, 90% or more, 91% or more, 92% or more, 93% or more, 94% or more, 95% or more, 96% or more, 97% or more, 98% or more, 99% or more, or 100% of a saturated dissolved oxygen during the preparation of the liquid medium.

14. The method for preparing a liquid medium according to any one of claims 1 to 13, wherein the amount of the solvent is 10 to 10000 L.

15. A liquid medium prepared by the preparation method according to any one of claims 1 to 14.

16. A method for culturing cells using the liquid medium according to claim 15.

17. The method for culturing cells according to claim 16, wherein the cells are cells that express a polypeptide.

18. The method for culturing cells according to claim 17, wherein the polypeptide is an antibody.

19. The method for culturing cells according to any one of claims 16 to 18, wherein the cells are animal cells.

20. The method for culturing cells according to claim 19, wherein the animal cells are Chinese hamster ovary cells.

21. The method for culturing cells according to any one of claims 16 to 20, wherein the cells are genetically modified cells.

22. A method for producing a polypeptide, comprising culturing cells that express a desired polypeptide in the liquid medium according to claim 15, producing and accumulating the polypeptide in a culture and collecting the polypeptide from the culture.

23. The method for producing a polypeptide according to claim 22, wherein the polypeptide is an antibody.

24. The method for producing a polypeptide according to claim 22 or 23, wherein the cells are animal cells.

25. The method for producing a polypeptide according to claim 24, wherein the animal cells are Chinese hamster ovary cells.

26. The method for producing a polypeptide according to any one of claims 22 to 25, wherein the cells are genetically modified cells.

27. A polypeptide produced using the production method according to any one of claims 22 to 26.

28. A method for controlling generation of a variant derived from a polypeptide, wherein the liquid medium according to claim 15 is used in a step of culturing cells that express the polypeptide.

29. The method for controlling the generation of a variant according to claim 28, wherein the polypeptide is an antibody.

30. The method for controlling the generation of a variant according to claim 29, wherein the variant is an antibody in which C-terminal proline is amidated.

31. The method for controlling the generation of a variant according to any one of claims 28 to 30, wherein the cells are animal cells.

32. The method for controlling the generation of a variant according to claim 31, wherein the animal cells are Chinese hamster ovary cells.

33. The method for controlling the generation of a variant according to any one of claims 28 to 32, wherein the cells are genetically modified cells.
